# NEUE EUROPÄISCHE PATENTSCHRIFT

(11) **EP 1 161 994 B2**
(45) Veröffentlichungstag und Bekanntmachung des Hinweises auf die Entscheidung über den Einspruch: **25.02.2009**
(45) Hinweis auf die Patenterteilung: 07.04.2004
(21) Anmeldenummer: 01111317.2
(22) Anmeldetag: 09.05.2001
(51) Int. Cl.: B01L 3/00, C12M 1/20, B01J 19/00

(54) **Mikrotiterplatte**
Micro-titer plate
Plaque de microtitrage

(30) Priorität: 08.06.2000 DE 10028536
(43) Veröffentlichungstag der Anmeldung: 12.12.2001
(73) Patentinhaber: EPPENDORF AG, 22339 Hamburg (DE)
(72) Erfinder: Gülzow, Nico, 22337 Hamburg (DE); Petersen, Bernd Ohm, 23627 Gross Grönau (DE)
(74) Vertreter: Hauck Patent- und Rechtsanwälte

(56) Entgegenhaltungen:
- EP-A- 0 106 662
- EP-A- 0 844 025
- WO-A-01/07160
- WO-A2-01/07160
- DE-A- 4 217 868
- DE-A- 4 321 033
- DE-A- 19 736 630
- GB-A- 2 268 187
- US-A- 5 710 381

## Beschreibung

Die Erfindung bezieht sich auf eine Mikrotiterplatte.

Mikrotiterplatten werden für die unterschiedlichsten mikrobiologischen, zellzüchterischen und immunologischen Arbeitsgänge benutzt. Insbesondere finden Mikrotiterplatten Anwendung für die PCR oder die Züchtung von Mikroorganismen oder Zellen.

Es sind bereits Mikrotiterplatten bekannt, die einen Rahmen mit einer Platte aufweisen, an der eine Vielzahl Gefäße fixiert ist, die einen von der Unterseite der Platte vorstehenden Aufnahmeabschnitt haben und durch Öffnungen von der Oberseite der Platte aus zugänglich sind. Die Gefäße werden auch als "Wells" bezeichnet. Die gängigen 96'er Mikrotiterplatten haben in Reihen und Spalten 8 x 12 = 96 Gefäße. Mehr und mehr werden aber auch Mikrotiterplatten mit einer größeren Anzahl Gefäße verwendet.

Einkomponentige Mikrotiterplatten aus Polystyrol sind für die PCR nicht geeignet, insbesondere weil die Erweichungstemperatur dieses Kunststoffes (etwa 85 °C) bei der PCR überschritten wird.

Einkomponentige Mikrotiterplatten aus Polypropylen sind grundsätzlich für die PCR verwendbar. Sie sind jedoch biegeweich, neigen zu Verzug, sind uneben und nur mit großen Toleranzen zu fertigen und unterliegen großen Toleranzschwankungen im Einsatz. Sie sind insbesondere für das Automatenhandling nicht besonders geeignet, weil ihre Weichheit das Greifen durch den Automaten erschwert. Ferner kann die geringe Maßhaltigkeit zur Folge haben, so daß die Dosiernadeln beim Einführen in die Gefäße Wandberührung haben. Außerdem ist aufgrund der Dickwandigkeit der Gefäße der Wärmetransfer in die Gefäße schlecht, was nachteilig für die Temperaturregelung und die Länge der Zykluszeiten bei der PCR ist. Zweikomponentige Mikrotiterplatten sind in der WO 01/07160 A2 (Veröffentlichungstag: 1.2.2001, Prioritätstage: 23.7.1999 und 19.7.2000, Anmeldetag: 20.7.2000) offenbart. Die Gefäße dieser Mikrotiterplatten haben einen vollständigen flachen Boden.

Insbesondere bei der Zucht von Mikroorganismen oder Zellen bedarf es einer ausreichenden Sauerstoffversorgung der Probe. Diese kann bei 96'er Mikrotiterplatten durch die relativ großen Öffnungen der Gefäße sichergestellt werden. Bei Mikrotiterplatten mit einer größeren Anzahl Gefäße ― beispielsweise 384 - kann die Sauerstoffzufuhr durch die verringerten Öffnungsquerschnitte jedoch stark beeinträchtigt sein. Außerdem wäre es zur Vermeidung von Querkontaminationen zwischen den Proben verschiedener Gefäße wünschenswert, eine Sauerstoffversorgung auch bei geschlossenen Öffnungen sicherzustellen.

Auch bei anderen Anwendungen von Mikrotiterplatten versucht man Querkontaminationen zu vermeiden. Hierzu gibt es Dichtfolien, die auf der Oberseite der Mikrotiterplatte aufgeschweißt werden und wieder abgelöst werden müssen, wenn auf den Inhalt der Gefäße Zugriff genommen werden soll. Darüber hinaus gibt es Gummimatten, die an der Unterseite Konusse aufweisen, um bei Auflage auf der Mikrotiterplatte dichtend in die Öffnungen der Gefäße einzugreifen. Ferner gibt es Kunststoffstreifen, die an der Unterseite mit Stopfen ausgeführt sind, um in die Öffnungen einer Gefäßreihe der Mikrotiterplatte eingedrückt zu werden.

Die bekannten Dichtungsmethoden sind kompliziert in der Anwendung werden erhöhten Dichtigkeitsanforderungen nicht gerecht.

Davon ausgehend liegt der Erfindung die Aufgabe zugrunde, eine Mikrotiterplatte mit günstigeren Anwendungseigenschaften zu schaffen.

Außerdem soll ein Verfahren zur Herstellung der Mikrotiterplatte zur Verfügung gestellt werden.

Die Aufgabe wird durch eine Mikrotiterplatte mit den Merkmalen mindestens eines der Ansprüche 1, 7, 13, 19, 24, 28 und 31 gelöst. Ferner wird sie durch ein Verfahren mit den Merkmalen mindestens eines der Ansprüche 42 und 44 gelöst.

Die erfindungsgemäße Mikrotiterplatte hat
- einen Rahmen aus einem steifen ersten Kunststoff, der eine Platte mit eine Vielzahl Löcher aufweist, und
- eine Vielzahl Gefäße aus einem zweiten Kunststoff, der hinsichtlich der Erweichungstemperatur für die PCR geeignet ist und/oder ein Silikon ist, die durch direktes Anspritzen an die Löcher fest mit der Platte verbunden sind, einen von der Unterseite der Platte vorstehenden Aufnahmeabschnitt haben und durch Öffnungen von der Oberseite der Platte aus zugänglich sind.

Der Rahmen der Mikrotiterplatte ist aufgrund seiner Steifigkeit besonders für das Automatenhandling geeignet. Vorzugsweise ist er am Rand mit einer von der Unterseite vorstehenden Einfassung versehen, welche die Stabilität erhöht, eine Standfläche bilden kann und eine Angriffsfläche für den Automaten bietet. Dabei kann der Rahmen besonders verzugsarm und toleranzarm gefertigt werden. Der erste Kunststoff kann ein amorpher oder aber auch ein teilkristalliner, hochgefüllter Kunststoff sein. Dabei kann es sich insbesondere um Polycarbonat handeln, welches an sich für die PCR bzw. die Sauerstoffzufuhr ungeeignet ist. Die Beschränkung dieses Kunststoffes auf dem Rahmen ermöglicht jedoch, seine vorteilhaften Eigenschaften auch bei Mikrotiterplatten für die PCR bzw. Sauerstoffversorgung von Proben nutzbar zu machen.

Die Gefäße bestehen aus einem anderen Kunststoff als der Rahmen. Es handelt sich dabei um einen für die PCR geeigneten und/oder eine Sauerstoffdurchlässigkeit aufweisenden zweiten Kunststoff. Die Eignung für die PCR kann insbesondere durch eine erhöhte Temperaturbeständigkeit (bis etwa 90 bis 95 °C) gegeben sein.

Ferner durch eine verringerte Affinität oder Neutralität des Kunststoffes zu DNA oder anderen Substanzen der PCR. Bevorzugt handelt es sich um einen weichen und/oder teilkristallinen Kunststoff. Vorzugsweise kann es sich bei dem zweiten Kunststoff um Polypropylen handeln.

Jedes Gefäß wird direkt an das ihm zugeordnete Loch angespritzt. Die Verbindung mit der Platte kann durch Kraftschluß und/oder durch Formschluß und/oder durch Stoffschluß erfolgen. Bevorzugt erfolgt sie durch Formschluß, indem die Gefäße an Löcher mit in Axialrichtung veränderlichem Querschnitt und/oder am Randbereich der Löcher auf zumindest einer Seite der Platte formschlüssig mit dieser verbunden sind.

Durch das direkte Anspritzen werden sehr geringe Fließwege des Material beim Spritzen ermöglicht, wodurch besonders geringe Wandstärken erreichbar sind, die vorzugsweise in den Bereich von etwa 0,05 bis 0,25 mm fallen, insbesondere etwa 0,1 mm betragen können. Dies begünstigt den Wärmetransfer. Dafür ist bevorzugt am Gefäßboden jedes Gefäßes ein Anspritzpunkt vorgesehen, von dem aus das Material einen ersten Wandabschnitt mit verringerter Wandstärke und einem oberen, mit der Platte verbundenen Wandabschnitt ausfüllt. Bevorzugt ist der obere Wandabschnitt als Kragen mit vergrößerter Wandstärke ausgeführt, was eine besonders toleranzarme Herstellung der Mikrotiterplatte ermöglicht.

Durch die Ausführung des Rahmens aus einem ersten Kunststoff und der Gefäße aus einem zweiten Kunststoff werden somit mittels Materialien, die den gewünschten Funktionen von Rahmen und Gefäßen entsprechen, bestmögliche Lösungen erreicht. Eine höhere Steifigkeit, bessere Planheit, geringere Verzugsneigung und kleinere Toleranzen werden durch ein amorphes, steifes und hochtemperaturfestes Material für den Rahmen erreicht. Die extreme Dünnwandigkeit für einen besseren Wärmetransfer wird durch direktes Anspritzen der Gefäße erreicht. Über die Gefäße wird kein Rahmen gefüllt, so daß der gesamte Druckgradient nur für jeweils ein Gefäß zur Verfügung steht. Die Gefäße können in weichen, für die PCR geeigneten Materialien gespritzt werden. Das Spritzen des Rahmens ist unkritisch. Dieser kann vorteilhaft mehrere randseitige Anspritzpunkte aufweisen (etwa vier bis sechs).

Im Hinblick auf eine erhöhte Sauerstoffdurchlässigkeit ist der zweite Kunststoff ein Silikon. Dabei kann es sich insbesondere um LSR (Liquid Silicon Rubber) handeln.

Nach dem erfindungsgemäßen Herstellungsverfahren werden Rahmen und Gefäße der Mikrotiterplatte in einem Mehrkomponentenspritzverfahren hergestellt. Im einfachsten Falle handelt es sich dabei um ein Zweikomponentenspritzverfahren oder "Twin-Shot"-Verfahren.

Für eine besonders toleranzarme Herstellung wird bevorzugt zunächst der Rahmen gespritzt und danach die Gefäße. Dies hat den Vorteil, daß der Rahmen zunächst eine gewisse Schrumpfung vollführen kann, bevor die Gefäße angespritzt werden. Der Zeitabstand für das Anspritzen der Gefäße vom Spritzen des Rahmens kann so gewählt werden, daß die Schrumpfung des Rahmens (durch Abkühlen) im wesentlichen vollständig erfolgt. Nach dem Anspritzen der Gefäße wird die Maßhaltigkeit der Mikrotiterplatte durch Schrumpfvorgänge praktisch nicht mehr beeinträchtigt. Insbesondere die Toleranz des Abstandes von Gefäß zu Gefäß kann so auf sehr geringe Werte begrenzt werden (etwa ± 0,15 mm). Dies begünstigt das Einführen von Dosiernadeln ohne Wandberührung.

Besonders vorteilhaft ist dabei, wenn der obere Wandbereich der Gefäße als Kragen mit vergrößerter Wandstärke ausgeführt ist, weil der Kragen beim Spritzen verbleibende Lagetoleranzen der Löcher ausgleichen kann.

Die Erfindung wird nachfolgend anhand der anliegenden Zeichnungen von Ausführungsbeispielen näher erläutert. In den Zeichnungen zeigen:
- Fig. 1: eine 96er-Mikroriterplatte mit Rahmen und Gefäßen aus verschiedenen Kunststoffen in der Draufsicht;
- Fig. 2: dieselbe Mikrotiterplatte in einer Perspektivansicht schräg von unten;
- Fig. 3: dieselbe Mikrotiterplatte in einem stark vergrößerten vertikalen Teilschnitt durch die Platte des Rahmens und ein Gefäß;
- Fig. 4: eine nicht beanspruchte 96er-Mikrotiterplatte mit integral mit integrierten ringförmigen Dichtungen in einer Perspektivansicht schräg von oben;
- Fig. 5: eine gegenüber der Ausführung von Fig. 4 durch Verbindungsstege zwischen den Dichtungen abgewandelte nicht beanspruchte Mikrotiterplatte in einer Teilperspektivansicht schräg von oben;
- Fig. 6: eine nicht beanspruchte Mikrotiterplatte mit einem Deckel mit integrierten pfropenförmigen Dichtungen in einem perspektivischen Teilschnitt.

Bei der nachfolgenden Beschreibung verschiedener Ausführungsbeispiele haben übereinstimmende Elemente dieselben Bezugsziffern. Die zugehörigen Beschreibung hat für sämtliche Ausführungsbeispiele Gültigkeit.

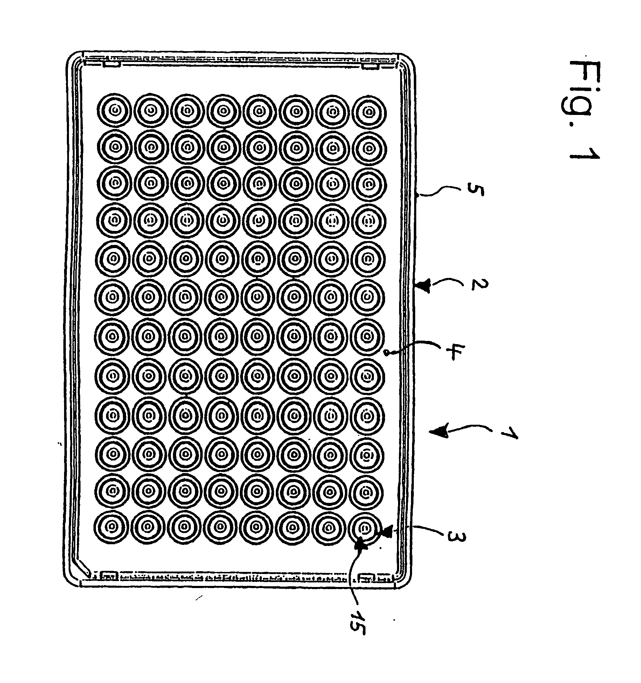

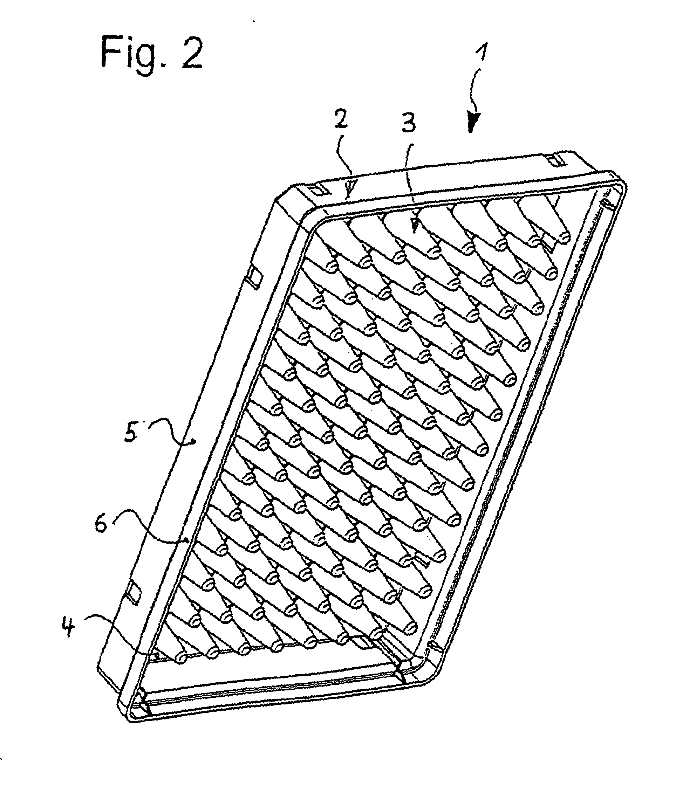

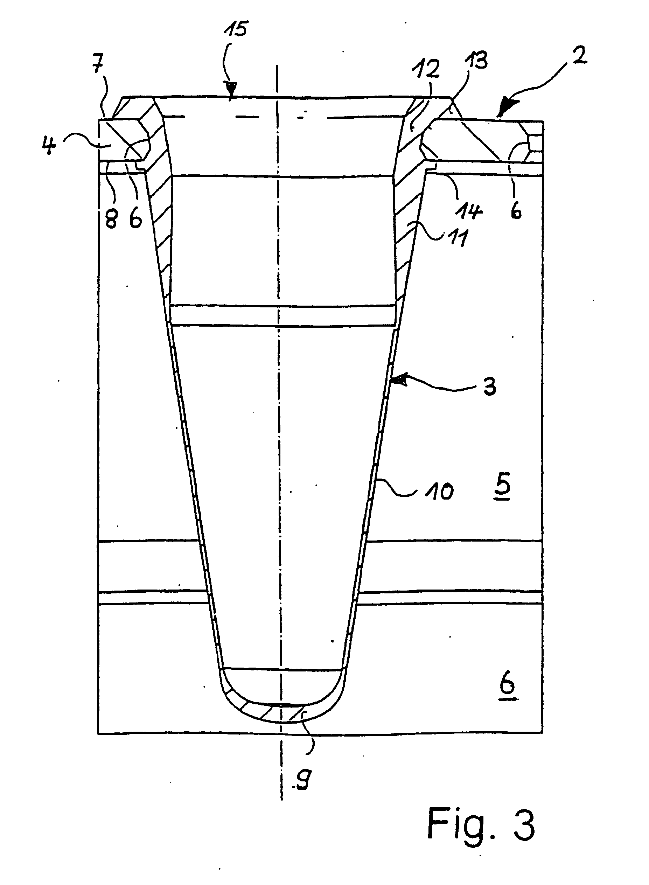

Gemäß Fig. 1 bis 3 besteht eine Mikrotiterplatte 1 aus einem Rahmen 2 und einer V ielzahl Gefäßen 3. In 8 Spalten und 12 Reihen sind insgesamt 96 Gefäße 3 vorhanden.

Der Rahmen 2 weist eine im wesentlichen rechteckige Platte 4 auf, um deren äußeren Rand eine Einfassung 5 umläuft, etwa senkrecht von der Unterseite der Platte 4 hinaussteht, und zwar über die Gefäße 3 hinaus. Unten hat die Einfassung 5 in bekannter Weise eine Aufweitung 6, die eine Stapelbarkeit auf der Oberseite einer entsprechenden Mikrotiterplatte 1 gewährleistet.

Der Rahmen 2 weist in der Platte 4 insgesamt 96 Löcher 6 auf. Diese haben jeweils einen Querschnittsverlauf, der sich zur Oberseite der Platte 7 hin in zwei Abschnitten unterschiedlicher Konizität und zur Unterseite 8 der Platte 4 in einem konischen Abschnitt erweitert.

Der Rahmen 2 ist in einem ersten Spritzschritt integral aus einem Kunststoff gespritzt, der ausgehärtet verhältnismäßig starr ist. Die Anspritzpunkte befinden sich am Rand des Rahmens 2, beispielsweise am unteren Rand der Einfassung 5.

Die Gefäße 3 haben unten einen napfförmigen Boden 9, an den ein kegelförmiger Wandabschnitt 10 sehr geringer Wandstärke (ca. 0,1 mm) angrenzt. Darüber befindet sich ein Wandabschnitt 11, dessen Wandstärke nach oben allmählich zunimmt. Außen weist er dieselbe Konizität wie der Wandabschnitt 10 auf. Innen ist er jedoch beinahe zylindrisch ausgeführt, wodurch sich ein etwa keilförmiger Querschnittsverlauf ergibt.

Der Wandabschnitt 11 endet in einem Kragen 12, der ebenfalls gegenüber dem Wandabschnitt 10 erheblich vergrößerte Wandstärke aufweist. Im Bereich des Kragens 12 sind die Gefäße 3 an die Platte 4 angespritzt. Dafür liegt der Kragen 12 außen am Innenumfang der Löcher 6 an. Ferner hat er an der Oberseite 7 und der Unterseite 8 der Platte 4 jeweils einen Überstand 13, 14, wodurch sich eine sichere Verbindung mit der Platte 4 ergibt.

Im Bereich des Kragens 12 haben die Gefäße 3 einen sich nach oben in zwei Abschnitten unterschiedlicher Konizität erweiternden Querschnitt. Von der Oberseite 7 der Platte 4 sind die Gefäße durch Öffnungen 15 zugänglich.

Sämtliche Gefäße sind gleichzeitig direkt an den Rahmen 1 bzw. an die Löcher 6 desselben angespritzt. Jedes Gefäß 6 hat einen eigenen zentralen Anspritzpunkt an der Unterseite des Bodens 9. Hierdurch werden kurze Spritzwege erreicht, die die besonders geringe Wandstärke im Wandabschnitt 10 ermöglicht. Als Material kommt im Beispiel Polypropylen oder LSR zum Einsatz, im Hinblick auf die PCR oder eine Sauerstoffzufuhr an eine Probe im Inneren des Gefäßes.

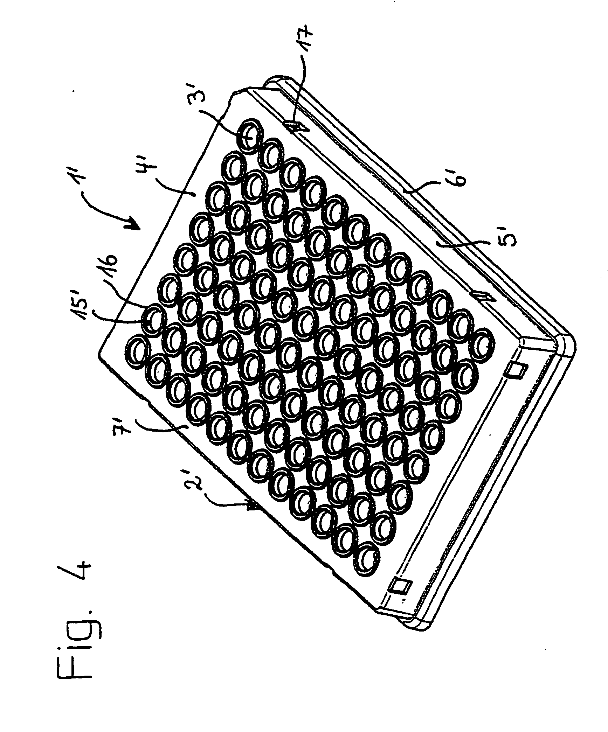

Fig. 4 zeigt eine Mikrotiterplatte 1', bei der in Abweichung von der zuvor erörterten der Rahmen 2' und die Gefäße 3' in bekannter Weise integral aus einem einzigen Kunststoff hergestellt sind. Die äußere Form der Mikrotiterplatte 1' entspricht dabei im wesentlichen dem vorangegangenen Beispiel, wobei allerdings die Gefäße 3' einen im wesentlichen gleichförmigen Wandstärkenverlauf haben und ohne Überstände mit der Platte 4' verschmolzen sind. Die Platte 4' ist randseitig in der bekannten Weise mit der Einfassung 5' verbunden, welche unten die Aufweitung 6' aufweist.

Die Gefäße 3' sind von oben durch Öffnungen 15' zugänglich, wobei um jede Öffnung eine ringförmige Dichtung 16 aus einem elastischen Material angeordnet ist. Im Beispiel handelt es sich dabei um einen Kunststoff, der in der Lage ist, sich stoffschlüssig mit dem Kunststoff der Mikrotiterplatte 1' zu verbinden.

Statt dessen kann eine formschlüssige Verbindung dadurch erzeugt werden, daß die Dichtung 16 in einer hinterschnittenen Nut in der Oberseite der Platte 4' eingebracht wird.

Vorzugsweise werden die Dichtungen 16 in einem Mehrkomponentenspritzverfahren fest mit der Mikrotiterplatte 1' verbunden.

Durch Aufsetzen eines - nicht gezeigten - Deckels aus einem starren Material ist es nun möglich, die Öffnungen 15' abdichtend zu verschließen. Der Deckel kann etwa die Abmessungen der Platte 4' haben. Vorzugsweise erfolgt eine Verrastung im Randbereich der Mikrotiterplatte 1'. Die Verrastung kann beispielsweise in den Ausnehmungen 17 erfolgen, welche die Einfassung 5' direkt unterhalb der Platte 4' aufweist.

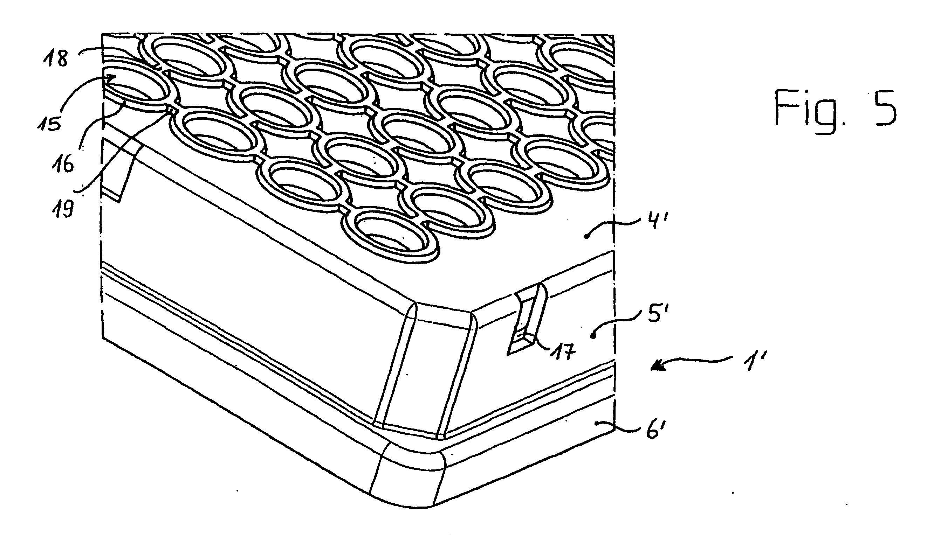

Die Ausführung von Fig. 5 unterscheidet sich von der vorbeschriebenen dadurch, daß die benachbarten, ringförmigen Dichtungen 16 miteinander durch geradlinige Stege 18, 19 verbunden sind, die sich in Reihen- und Spaltenrichtung erstrecken. Dies kann insbesondere aus fertigungstechnischen Gründen vorteilhaft sein, aber auch aus Gründen der festen Verbindung der Dichtungen mit der Mikrotiterplatte 1' oder zur zusätzlichen Abdichtung.

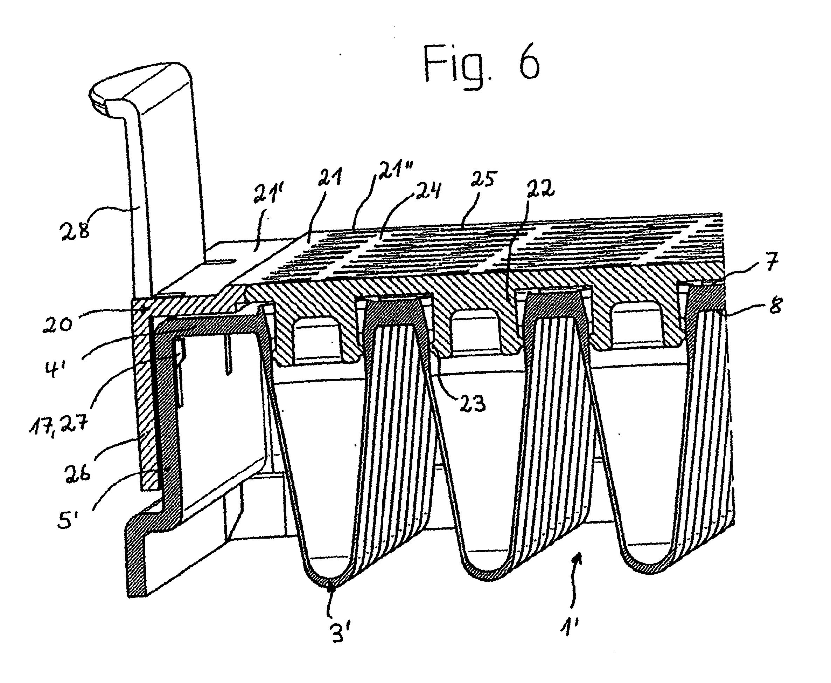

In der Fig. 6 ist eine Mikrotiterplatte 1' gezeigt, die entsprechend der von Fig. 4 aus nur einem Material hergestellt ist. Es fehlen allerdings die ringförmigen Dichtungen 16. Auf der Platte 4' der Mikrotiterplatte 1' sitzt ein Deckel 20. Dieser hat eine Platte 21, die im wesentlichen dieselbe Kontur wie die Platte 4' hat. Die Platte 21 ist in Randbereichen 21' auf der Oberseite der Platte 4' abgestützt. In einem Bereich 21" zwischen den Randbereichen 21' ist sie um einen kleinen Spalt von der Platte 4' beabstandet. Dies ermöglicht ein Aufsetzen auf herkömmliche Mikrotiterplatten, die Dichtungskragen an der Oberseite der Halteplatte 4' aufweisen.

In dem Bereich 21" hat die Platte 21 pfropfenförmige Dichtungen 22, die von ihrer Unterseite vorstehen. Diese pfropfenförmigen Dichtungen 22 haben an ihrem Außenumfang einen umlaufenden Dichtwulst 23.

Jeder Öffnung 15' der Gefäße 3' ist eine Dichtung 22 zugeordnet. Dabei greifen die Dichtungen 22 so in die Öffnungen 15' ein, daß sie mit ihren Dichtwulsten 23 an der Innenwand der Gefäße 3' abdichtend anliegen.

Die Dichtungen 22 sind in entsprechenden Ausnehmungen der Platte 21 angeordnet. Sie sind miteinander durch kurze Stege 24, 25 verbunden, die sich in Reihen- und Spaltenrichtung erstrecken.

Am Rand der Platte 21 stehen von deren Unterseite Einfassungen 26 vor, von denen nach innen Rastvorsprünge 27 vorstehen, welche in die Ausnehmungen 17 der Mikrotiterplatte 1' einrastbar sind. Nach oben stehen von den Einfassungen 26 Handgriffe 28 vor. Diese erleichtern ein Ergreifen des Deckels 20. Außerdem ist es durch Schwenken der Handgriffe 28 möglich, die Verrastung zwischen den Rastvorsprüngen 27 und den Ausnehmungen 17 aufzulösen, weil die Einfassungen 26 mitgeschwenkt werden.

Der Deckel 21 mit den Dichtungen 22 wird vorzugsweise ebenfalls in einem Mehrkomponentenspritzverfahren hergestellt.

## Patentansprüche

1. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der die Gefäße (3) durch Anspritzen an Löcher (6) mit in Axialrichtung veränderlichem Querschnitt formschlüssig mit der Platte (4) verbunden sind.

2. Mikrotiterplatte nach Anspruch 1, bei der die Gefäße (3) durch Anspritzen an den Randbereich der Löcher (6) auf zumindest einer Seite (7, 8) der Platte (4) formschlüssig mit dieser verbunden sind.

3. Mikrotiterplatte nach Anspruch 1 oder 2, bei der die Gefäße (3) Anspritzpunkte am Gefäßboden (9) aufweisen.

4. Mikrotiterplatte nach einem der Ansprüche 1 bis 3, bei der die Gefäße (3) als oberen mit der Platte (4) verbundenen Wandabschnitt (12) einen Kragen mit vergrößerter Wandstärke haben.

5. Mikrotiterplatte nach einem der Ansprüche 1 bis 4, bei der die Gefäße (3) Wandabschnitte (10), die im wesentlichen konisch sind, mit geringer Wandstärke haben, oder bei der die Gefäße (3) Wandabschnitte, die im wesentlichen konisch sind, mit geringer Wandstärke mit einem daran angrenzenden, eine sich nach oben allmählich vergrößernde Wandstärke aufweisenden Wandabschnitt (11) haben.

6. Mikrotiterplatte nach einem der Ansprüche 1 bis 5, bei der die Außenseite des Bodens (9) der Gefäße (3) nicht vollständig flach ist.

7. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der die Gefäße (3) durch Anspritzen an den Randbereich der Löcher (6) auf der Unterseite (8) der Platte (4) formschlüssig mit dieser verbunden sind.

8. Mikrotiterplatte nach Anspruch 7, bei der die Gefäße (3) durch Anspritzen an den Randbereich der Löcher (6) auf der Oberseite (7) der Platte (4) formschlüssig mit dieser verbunden sind.

9. Mikrotiterplatte nach Anspruch 7 oder 8, bei der die Gefäße (3) Anspritzpunkte am Gefäßboden (9) aufweisen.

10. Mikrotiterplatte nach einem der Ansprüche 7 bis 9, bei der die Gefäße (3) als oberen mit der Platte (4) verbundenen Wandabschnitt (12) einen Kragen mit vergrößerter Wandstärke haben.

11. Mikrotiterplatte nach einem der Ansprüche 7 bis 10 bei der die Gefäße (3) Wandabschnitte (10), die im wesentlichen konisch sind, mit geringer Wandstärke haben, oder bei der die Gefäße (3) Wandabschnitte, die im wesentlichen konisch sind, mit geringer Wandstärke mit einem daran angrenzenden, eine sich nach oben allmählich vergrößernde Wandstärke aufweisenden Wandabschnitt (11) haben.

12. Mikrotiterplatte nach einem der Ansprüche 7 bis 11, bei der die Außenseite des Bodens (9) der Gefäße (3) nicht vollständig flach ist.

13. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der die Gefäße (3) Anspritzpunkte am Gefäßboden (9) aufweisen.

14. Mikrotiterplatte nach Anspruch 13, bei der die Gefäße (3) formschlüssig mit der Platte (4) verbunden sind.

15. Mikrotiterplatte nach Anspruch 13 oder 14, bei der die Gefäße (3) durch Anspritzen an den Randbereich der Löcher (6) auf zumindest einer Seite (7, 8) der Platte (4) formschlüssig mit dieser verbunden sind.

16. Mikrotiterplatte nach einem der Ansprüche 13 bis 15, bei der die Gefäße (3) als oberen mit der Platte (4) verbundenen Querschnitt (12) einen Kragen mit vergrößerter Wandstärke halben.

17. Mikrotiterplatte nach einem der Ansprüche 13 bis 16 bei der die Gefäße (3) Wandabschnitte (10), die im wesentlichen konisch sind, mit geringer Wandstärke haben, oder bei der die Gefäße (3) Wandabschnitte, die im wesentlichen konisch sind, mit geringer Wandstärke mit einem daran angrenzenden, eine sich nach oben allmählich vergrößernde Wandstärke aufweisenden Wandabschnitt (11) haben.

18. Mikrotiterplatte nach einem der Ansprüche 13 bis 17, bei der die Außenseite des Bodens (9) der Gefäße (3) nicht vollständig flach ist.

19. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direkte Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der die Gefäße (3) als oberen mit der Platte (4) verbundenen Wandabschnitt (12) einen außen am Innenumfang der Löcher (6) anliegenden Kragen (12) mit vergrößerter Wandstärke haben.

20. Mikrotiterplatte nach Anspruch 19, bei der die Gefäße (3) formschlüssig mit der Platte (4) verbunden sind.

21. Mikrotiterplatte nach Anspruch 19 oder 20, bei der die Gefäße (3) durch Anspritzen an den. Randbereich der Löcher (6) auf zumindest einer Seite (7, 8) der Platte (4) formschlüssig mit dieser verbunden sind.

22. Mikrotiterplatte nach einem der Ansprüche 10 bis 21 bei der die Gefäße (3) Wandabschnitte (10), die im wesentlichen konisch sind, mit geringer Wandstärke haben, oder bei der die Gefäße (3) Wandabschnitte, die im wesentlichen konisch sind, mit geringer Wandstärke mit einem daran angrenzenden, eine sich nach oben allmählich vergrößernde Wandstärke aufweisenden Wandabschnitt (11) haben.

23. Mikrotiterplatte nach einem der Ansprüche 19 bis 22, bei der die Außenseite des Bodens (9) der Gefäße (3) nicht vollständig flach ist.

24. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der die Gefäße (3) einen im wesentlichen konischen Wandabschnitt (10) mit geringer Wandstärke haben und bei einem daran angrenzenden Wandabschnitt (11) eine sich nach oben allmählich vergrößernde Wandstärke aufweisen.

25. Mikrotiterplatte nach Anspruch 24, bei der die Gefäße (3) formschlüssig mit der Platte (4) verbunden sind.

26. Mikrotiterplatte nach Anspruch 24 oder 25, bei der die Gefäße (3) durch Anspritzen an den Randbereich der Löcher (6) auf zumindest einer Seite (7, 8) der Platte (4) formschlüssig mit dieser verbunden sind.

27. Mikrotiterplatte nach einem der Ansprüche 24 bis 26, bei der die Außenseite des Bodens (9) der Gefäße (3) nicht vollständig flach ist.

28. Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, und
- bei der eine Außenseite eines Bodens (9) zumindest eines der Gefäße (3) nicht vollständig flach ist.

29. Mikrotiterplatte nach Anspruch 28, bei der die Gefäße (3) formschlüssig mit der Platte (4) verbunden sind.

30. Mikrotiterplatte nach Anspruch 28 oder 29, bei der die Gefäße (3) durch Anspritzen an den Randbereich der Löcher (6) auf zumindest einer Seite (7, 8) der Platte (4) formschlüssig mit dieser verbunden sind.

31. Mikrotiterplatte mit
- einem Rahmen (2) aus einem steifen ersten Kunststoff, der eine Platte (4) mit einer Vielzahl Löcher (6) aufweist, und
- einer Vielzahl Gefäße (3) aus einem zweiten Kunststoff, der ein Silikon ist, die durch direkte Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind.

32. Mikrotiterplatte nach einem der Ansprüche 1 bis 31, bei der die Gefäße (3) kranschlüssig und/oder stoffschlüssig mit der Platte (4) verbunden sind.

33. Mikrotiterplatte nach einem der Ansprüche 1 bis 32, bei der die Gefäße (3) angrenzend an einen Gefäßboden (9) einen Wandabschnitt (10) mit sehr geringer Wandstärke haben und mit einem oberen Wandabschnitt (12) mit der Platte (4) verbunden sind.

34. Mikrotiterplatte nach einem der Ansprüche 1 bis 33, bei der die Gefäße (3) zumindest in einem Wandabschnitt (10) eine Wandstärke von etwa 0,05 bis 0,25 mm aufweisen.

35. Mikrotiterplatte nach einem der Ansprüche 1 bis 34, bei der die Gefäße (3) einen im wesentlichen napfförmigen Boden (9) haben.

36. Mikrotiterplatte nach einem der Ansprüche 1 bis 35, bei der der Rahmen (2) am Rand der Platte (4) eine von deren Unterseite (8) vorstehende Einfassung (5) aufweist.

37. Mikrotiterplatte nach einem der Ansprüche 1 bis 36, bei der der Rahmen (2) mehrere randseitige Anspritzpunkte aufweist.

38. Milcrotiterplatte nach einem der Ansprüche 1 bis 37, bei der der Rahmen (2) aus einem amorphen Kunststoff oder aus einem teilkristallinen, hochgefüllten Kunststoffbesteht.

39. Mikrotiterplatte nach einem der Ansprüche 1 bis 38, bei der der Rahmen (2) aus Polycarbonat besteht.

40. Mikrotiterplatte nach einem der Ansprüche 1 bis 39, bei der die Gefäße (3) aus einem weichen und/oder teilkristallinen Kunststoff bestehen.

41. Mikrotiterplatte nach einem der Ansprüche 1 bis 40, bei der die Gefäße aus Polypropylen oder LSR bestehen.

42. Verfahren zum Herstellen einer Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, insbesondere nach einem der Ansprüche 1 bis 41,
- bei dem der Rahmen (2) und die Gefäße (3) in einem Mehrkomponentenspritzverfahren hergestellt werden,
- bei dem zunächst der Rahmen (2) und danach die Gefäße (3) gespritzt werden und
- bei dem die Gefäße (3) von jeweils einem eigenen Anspritzpunkt von ihrem Boden (9) aus gespritzt werden.

43. Verfahren nach Anspruch 42, bei dem in einem das Schrumpfen des Rahmens (2) im wesentlichen vollständig gewährleistenden Zeitabstand vom Spritzen des Rahmens (2) die Gefäße (3) gespritzt werden.

44. Verfahren zum Herstellen einer Mikrotiterplatte mit
- einem Rahmen (2) aus einem steifen ersten Kunststoff, der eine Platte (4) mit einer Vielzahl Löcher (6) aufweist,
- einer Vielzahl Gefäße (3) aus einem zweiten Kunststoff, der hinsichtlich der Erweichungstemperatur für die PCR geeignet ist und/oder ein Silikon ist, die durch direktes Anspritzen an die Löcher (6) fest mit der Platte (4) verbunden sind, einen von der Unterseite (8) der Platte (4) vorstehenden Aufnahmeabschnitt (9, 10, 11) haben und durch Öffnungen (15) von der Oberseite (7) der Platte aus zugänglich sind, insbesondere nach einem der Ansprüche 1 bis 41,
- bei dem der Rahmen (2) und die Gefäße (3) in einem Mehrkomponentenspritzverfahren hergestellt werden,
- bei dem zunächst der Rahmen (2) und danach die Gefäße (3) gespritzt werden und
- bei dem in einem das Schrumpfen des Rahmens (2) im wesentlichen vollständig gewährleistenden Zeitabstand vom Spritzen des Rahmens (2) die Gefäße (3) gespritzt werden.

45. Verfahren nach Anspruch 44, bei dem die Gefäße von jeweils einem eigenen Anspritzpunkt von ihrem Boden (9) aus gespritzt werden.

46. Verfahren nach einem der Ansprüche 42 bis 45, bei dem der Rahmen (2) von mehreren Anspritzpunkten im Randbereich aus gespritzt wird.

## Claims

1. A microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by directly molding them to the holes (6), have a receiving portion (9, 10, 11) protruding from the lower side (8) of the plate (4), and are accessible from the upper surface (7) of the plate through apertures (15),
- wherein the vessels (3) are positively joined to the plate (4) by being molded to holes (6) of an axially variable cross-section.

2. The microtitration plate according to claim 1 wherein the vessels (3) are positively connected to the plate (4) on at least one side (7, 8) thereof by being molded to the marginal area of the holes (6).

3. The microtitration plate according to claim 1 or 2 wherein the vessels (3) have molding points at the vessel bottoms.

4. The microtitration plate according to any one of claims 1 to 3 wherein the vessels (3) have a collar of an increased wall thickness, as an upper wall portion (12) joined to the plate (4).

5. The microtitration plate according to any one of claims 1 to 4 wherein the vessels (3) have wall portions (10), which substantially are conical, of a small wall thickness or wherein the vessels (3) have wall portions, which substantially are conical, of a small wall thickness with a wall portion (11) adjacent thereto the wall thickness of which gradually increases upwardly.

6. The microtitration plate according to any one of claims 1 to 5 wherein the outside of the bottoms (9) of the vessels (3) is incompletely flat.

7. The microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15),
- wherein the vessels (3) are positively joined to the plate (4) by being molded to the marginal area of the holes (6) at the lower side (8) thereof.

8. The microtitration plate according to claim 7 wherein the vessels (3) are positively joined to the plate (4) by being molded to the marginal area of the holes (6) at the upper side (7) thereof.

9. The microtitration plate according to claim 7 or 8 wherein the vessels (3) have molding points at the vessel bottoms (9).

10. The microtitration plate according to any one of claims 7 to 9 wherein the vessels (3) have a collar of an increased wall thickness, as an upper wall portion (12) joined to the plate (4).

11. The microtitration plate according to any one of claims 7 to 10 wherein the vessels (3) have wall portions (10), which substantially are conical, of a small wall thickness or wherein the vessels (3) have wall portions, which substantially are conical, of a small wall thickness with a wall portion (11) adjacent thereto the wall thickness of which gradually increases upwardly.

12. The microtitration plate according to any one of claims 7 to 11 wherein the outside of the bottoms (9) of the vessels (3) is incompletely flat.

13. The microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15),
- wherein the vessels (3) have molding points at the vessel bottoms (9).

14. The microtitration plate according to claim 13 wherein the wherein the vessels (3) are positively joined to the plate (4).

15. The microtitration plate according to claim 13 or 14 wherein the vessels (3) are positively joined to the plate (4) at least on one side (7, 8) thereof by being molded to the marginal area of the holes (6).

16. The microtitration plate according to any one of claims 13 to 15 wherein the vessels (3) have a collar of an increased wall thickness, as an upper wall portion joined to the plate (4).

17. The microtitration plate according to any one of claims 13 to 16 wherein the vessels (3) have wall portions (10), which substantially are conical, of a small wall thickness or wherein the vessels (3) have wall portions, which substantially are conical, of a small wall thickness with a wall portion (11) adjacent thereto the wall thickness of which gradually increases upwardly.

18. The microtitration plate according to any one of claims 13 to 17 wherein the outside of the bottoms (9) of the vessels (3) is incompletely flat.

19. The microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15), and
- wherein the vessels (3) have a collar (12) of an increased wall thickness that externally bears on the inner circumference of the holes (6), as an upper wall portion (12) joined to the plate (4).

20. The microtitration plate according to claim 19 wherein the wherein the vessels (3) are positively joined to the plate (4).

21. The microtitration plate according to claim 19 or 20 wherein the vessels (3) are positively joined to the plate (4) at least on one side (7, 8) thereof by being molded to the marginal area of the holes (6).

22. The microtitration plate according to any one of claims 19 to 21 wherein the vessels (3) have wall portions (10), which substantially are conical, of a small wall thickness or wherein the vessels (3) have wall portions, which substantially are conical, of a small wall thickness with a wall portion (11) adjacent thereto the wall thickness of which gradually increases upwardly.

23. The microtitration plate according to any one of claims 19 to 22 wherein the outside of the bottom (9) of the vessels (3) is incompletely flat.

24. The microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15),
- wherein the vessels (3) have a substantially conical wall portion (10) of a small wall thickness with a wall portion (11) adjacent thereto the wall thickness of which gradually increases upwardly.

25. The microtitration plate according to claim 24 wherein the vessels (3) are positively are positively joined to the plate (4).

26. The microtitration plate according to claim 24 or 25 wherein the vessels (3) are positively joined to the plate (4) at least on one side (7, 8) thereof by being molded to the marginal area of the holes (6).

27. The microtitration plate according to any one of claims 24 to 26 wherein the outside of the bottom (9) of the vessels (3) is incompletely flat.

28. The microtitration plate, comprising
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15), and
- wherein one outside of a bottom (9) of at least one of the vessels (3) is incompletely flat.

29. The microtitration plate according to claim 28 wherein the vessels (3) are positively are positively joined to the plate (4).

30. The microtitration plate according to claim 28 or 29 wherein the vessels (3) are positively joined to the plate (4) at least on one side (7, 8) thereof by being molded to the marginal area of the holes (6).

31. The microtitration plate, comprising
- a frame (2) made of a first stiff plastic which has a plate (4) with a multiplicity of holes (6),
- a multiplicity of vessels (3) made of a second plastic which is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15).

32. The microtitration plate according to any one of claims 1 to 31 wherein the vessels (3) are joined to the plate (4) positively and/or in a material fit.

33. The microtitration plate according to any one of claims 1 to 32 wherein the vessels (3) have a wall portion (10) of a very small wall thickness adjacent to a vessel bottom (9), and are joined to the plate (4) by an upper wall portion (12).

34. The microtitration plate according to any one of claims 1 to 33 wherein the vessels (3) are of a wall thickness of from about 0.05 to 0.25 mm at least in one wall portion (10).

35. The microtitration plate according to any one of claims 1 to 34 wherein the vessels (3) have a substantially cup-shaped bottom (9).

36. The microtitration plate according to any one of claims 1 to 35 wherein the frame (2) has a bordering (5) protruding from the lower side (8) thereof at the edge of the plate (4).

37. The microtitration plate according to any one of claims 1 to 36 wherein the frame (2) has several edge-sided molding points.

38. The microtitration plate according to any one of claims 1 to 37 wherein the frame (2) is made of an amorphous plastic or a partially crystalline, heavily filled plastic.

39. The microtitration plate according to any one of claims 1 to 38 wherein the frame (2) is made of polycarbonate.

40. The microtitration plate according to any one of claims 1 to 39 wherein the vessels (3) are made of a soft and/or partially crystalline plastic.

41. The microtitration plate according to any one of claims 1 to 40 wherein the vessels (3) are made of polypropylene or LSR.

42. A method for manufacturing a microtitration plate, comprising:
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15), particularly according to any one of claims 1 to 41,
- wherein the frame (2) and the vessels (3) are manufactured by a multicomponent molding technique,
- in which the frame (2) is molded first and the vessels (3) are molded subsequently, and
- in which the vessels (3) are molded each from a molding point of their own, starting from their bottom (9).

43. The process according to claim 42 wherein the vessels (3) are molded at such interval from the molding of the frame (2) as substantially ensures the shrinking of the frame (2) completely.

44. A method for manufacturing a microtitration plate, comprising:
- a frame (2) made of a first stiff plastic which has a plate (4) with a multiplicity of holes (6),
- a multiplicity of vessels (3) made of a second plastic which is suited for the PCR with regard to its softening temperature and/or is a silicone, which are fixedly connected to the plate (4) by being directly molded to the holes (6), have a receiving portion (9, 10, 11) protruding from the lower side (8) of the plate (4), and are accessible from the upper surface (7) of the plate through apertures (15), particularly according to any one of claims 1 to 41,
- wherein the frame (2) and the vessels (3) are manufactured in a multicomponent molding technique,
- in which the frame (2) is molded initially and the vessels (3) are molded subsequently, and
- in which the vessels (3) are molded at such interval from the molding of the frame (2) as substantially ensures the shrinking of the frame (2) completely.

45. A method according to claim 44 wherein the vessels (3) are molded each from a molding point of their own, starting from their bottom (9).

46. A method according to any one of claims 42 to 45 wherein the frame (2) is molded from several molding points in the marginal area.

## Revendications

1. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- pour laquelle les récipients (3) sont reliés par conjugaison de forme à la plaque (4) par moulage par injection sur des orifices (6) dont la section est variable dans la direction axiale.

2. Plaque de microtitration selon la revendication 1, dans laquelle les récipients (3) sont reliés par conjugaison de forme à la plaque (4) par moulage par injection sur le bord des orifices (6), sur une face (7, 8) au moins de celle-ci.

3. Plaque de microtitration selon la revendication 1 ou 2, dans laquelle les récipients (3) présentent des points d'injection sur les fonds de récipient (9).

4. Plaque de microtitration selon l'une quelconque des revendications 1 à 3, dans laquelle les récipients (3) possèdent en tant que partie de paroi supérieure (12) reliée à la plaque (4) une collerette présentant une épaisseur de paroi augmentée.

5. Plaque de microtitration selon l'une quelconque des revendications 1 à 4, dans laquelle les récipients (3) ont des parties de paroi (10) qui sont sensiblement coniques avec une faible épaisseur de paroi, ou dans laquelle les récipients (3) ont des parties de paroi qui sont sensiblement coniques avec une faible épaisseur de paroi avec une partie de paroi (11) adjacente présentant une épaisseur de paroi progressivement croissante vers le haut.

6. Plaque de microtitration selon l'une quelconque des revendications 1 à 5, dans laquelle la face extérieure du fond (9) des récipients (3) n'est pas complètement plate.

7. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- dans laquelle les récipients (3) sont reliés par conjugaison de forme à la plaque (4) par injection sur le bord des orifices (6), sur la face inférieure (8) de la plaque.

8. Plaque de microtitration selon la revendication 7, dans laquelle les récipients (3) sont reliés par conjugaison de forme à la plaque (4) par injection sur le bord des orifices (6), sur la face supérieure (7) de la plaque.

9. Plaque de microtitration selon la revendication 7 ou 8, dans laquelle les récipients (3) présentent des points d'injection au niveau du fond de récipient (9).

10. Plaque de microtitration selon l'une quelconque des revendications 7 à 9, dans laquelle les récipients (3) possèdent en tant que partie supérieure de paroi (12) reliée à la plaque (4) une collerette avec une épaisseur de paroi augmentée.

11. Plaque de microtitration selon l'une quelconque des revendications 7 à 10, dans laquelle les récipients (3) ont des parties de paroi (10) qui sont sensiblement coniques avec une faible épaisseur de paroi, ou dans laquelle les récipients (3) ont des parties de paroi qui sont sensiblement coniques avec une faible épaisseur de paroi avec une partie de paroi (11) adjacente présentant une épaisseur de paroi progressivement croissante vers le haut.

12. Plaque de microtitration selon l'une quelconque des revendications 7 à 11, dans laquelle la face extérieure du fond (9) des récipients (3) n'est pas complètement plate.

13. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- dans laquelle les récipients (3) présentent des points d'injection sur les fonds de récipient (9).

14. Plaque de microtitration la revendication 13, dans laquelle les récipients (3) sont reliés par conjugaison de forme à la plaque (4).

15. Plaque de microtitration selon la revendication 13 ou 14, dans laquelle les récipients (3) sont reliés par conjugaison de forme à la plaque (4) par moulage par injection sur le bord des orifices (6), sur une face (7, 8) au moins de celle-ci.

16. Plaque de microtitration selon l'une quelconque des revendications 13 à 15, dans laquelle les récipients (3) possèdent en tant que partie de paroi supérieure (12) reliée à la plaque (4) une collerette présentant une épaisseur de paroi augmentée.

17. Plaque de microtitration selon l'une quelconque des revendications 13 à 16, dans laquelle les récipients (3) ont des parties de paroi (10) qui sont sensiblement coniques avec une faible épaisseur de paroi, ou dans laquelle les récipients (3) ont des parties de paroi qui sont sensiblement coniques avec une faible épaisseur de paroi avec une partie de paroi (11) adjacente présentant une épaisseur de paroi progressivement croissante vers le haut.

18. Plaque de microtitration selon l'une quelconque des revendications 13 à 17, dans laquelle la face extérieure du fond (9) des récipients (3) n'est pas complètement plate.

19. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- dans laquelle les récipients (3) possèdent en tant que partie de paroi supérieure (12) reliée à la plaque (4) une collerette présentant une épaisseur de paroi augmentée.

20. Plaque de microtitration selon la revendication 19, dans laquelle les récipients (3) sont reliés par conjugaison de forme à la plaque (4).

21. Plaque de microtitration selon la revendication 19 ou 20, dans laquelle les récipients (3) sont reliés par conjugaison de forme à la plaque (4) par moulage par injection sur le bord des orifices (6), sur une face (7, 8) au moins de celle-ci.

22. Plaque de microtitration selon l'une quelconque des revendications 19 à 21, dans laquelle les récipients (3) ont des parties de paroi (10) qui sont sensiblement coniques avec une faible épaisseur de paroi, ou dans laquelle les récipients (3) ont des parties de paroi qui sont sensiblement coniques avec une faible épaisseur de paroi avec une partie de paroi (11) adjacente présentant une épaisseur de paroi progressivement croissante vers le haut.

23. Plaque de microtitration selon l'une quelconque des revendications 19 à 22, dans laquelle la face extérieure du fond (9) des récipients (3) n'est pas complètement plate.

24. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- dans laquelle les récipients (3) ont une partie de paroi (10) qui est sensiblement conique avec une faible épaisseur de paroi et présentent sur une partie de paroi (11) adjacente une épaisseur de paroi progressivement croissante vers le haut.

25. Plaque de microtitration selon la revendication 24, dans laquelle les récipients (3) sont reliés par conjugaison de forme à la plaque (4).

26. Plaque de microtitration selon la revendication 24 ou 25, dans laquelle les récipients (3) sont reliés par conjugaison de forme à la plaque (4) par moulage par injection sur le bord des orifices (6), sur une face (7, 8) au moins de celle-ci.

27. Plaque de microtitration selon l'une quelconque des revendications 24 à 26, dans laquelle la face extérieure du fond (9) des récipients (3) n'est pas complètement plate.

28. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, et
- dans laquelle une face extérieure d'un fond (9) d'au moins un des récipients (3) n'est pas complètement plate.

29. Plaque de microtitration selon la revendication 28, dans laquelle les récipients (3) sont reliés par conjugaison de forme à la plaque (4).

30. Plaque de microtitration selon la revendication 28 ou 29, dans laquelle les récipients (3) sont reliés par conjugaison de forme à la plaque (4) par moulage par injection sur le bord des orifices (6), sur une face (7, 8) au moins de celle-ci.

31. Plaque de microtitration, comportant
- un cadre (2) composé d'un premier plastique rigide, qui comporte une plaque (4) munie d'une pluralité d'orifices (6),
- une pluralité de récipients (3) composés d'un second plastique qui est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque.

32. Plaque de microtitration selon l'une quelconque des revendications 1 à 31, dans laquelle les récipients (3) sont reliés à la plaque (4) par adhérence et/ou par continuité de matière.

33. Plaque de microtitration selon l'une quelconque des revendications 1 à 32, dans laquelle les récipients (3) ont une partie de paroi (10) adjacente à un fond de récipient (9) avec une très faible épaisseur de paroi et sont reliés par une partie de paroi supérieure (12) à la plaque (4).

34. Plaque de microtitration selon l'une quelconque des revendications 1 à 33, dans laquelle les récipients (3) présentent au moins dans une partie de paroi (10) une épaisseur de paroi d'environ 0,05 à 0,25 mm.

35. Plaque de microtitration selon l'une quelconque des revendications 1 à 34, dans laquelle les récipients (3) ont un fond (9) sensiblement de la forme d'une cuvette

36. Plaque de microtitration selon l'une quelconque des revendications 1 à 35, dans laquelle le cadre (2) comporte au bord de la plaque (4) une bordure (5) faisant saillie depuis la face inférieure (8) de la plaque.

37. Plaque de microtitration selon l'une quelconque des revendications 1 à 36, dans laquelle le cadre (2) présente plusieurs points d'injection du côté du bord.

38. Plaque de microtitration selon l'une quelconque des revendications 1 à 37, dans laquelle le cadre (2) se compose d'un plastique amorphe ou d'un plastique semi-cristallin très chargé.

39. Plaque de microtitration selon l'une quelconque des revendications 1 à 38, dans laquelle le cadre (2) se compose de polycarbonate.

40. Plaque de microtitration selon l'une quelconque des revendications 1 à 39, dans laquelle les récipients (3) se composent d'un plastique mou et/ou semi-cristallin.

41. Plaque de microtitration selon l'une quelconque des revendications 1 à 40, dans laquelle les récipients se composent de polypropylène ou de caoutchouc de silicone liquide.

42. Procédé de fabrication d'une plaque de microtitration comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, en particulier selon l'une quelconque des revendications 1 à 41,
- dans lequel le cadre (2) et les récipients (3) sont fabriqués dans un procédé de moulage par injection de plusieurs composants,
- dans lequel le cadre (2) d'abord, puis les récipients (3) sont moulés par injection, et
- dans lequel les récipients (3) sont moulés chacun par injection depuis un point d'injection propre de leur fond (9).

43. Procédé selon la revendication 42, dans lequel les récipients (3) sont injectés à la suite de l'injection du cadre (2) après écoulement d'un temps assurant à peu près complètement la contraction du cadre (2).

44. Procédé de fabrication d'une plaque de microtitration comportant
- un cadre (2) composé d'un premier plastique rigide, qui comporte une plaque (4) munie d'une pluralité d'orifices (6),
- une pluralité de récipients (3) composés d'un second plastique qui est approprié pour la PCR en ce qui concerne le point de ramollissement et/ou est une silicone, lesquels récipients sont reliés de façon fixe à la plaque (4) par moulage par injection directe sur les orifices (6), ont une partie de réception (9, 10, 11) saillant par rapport à la face inférieure (8) de la plaque (4) et sont accessibles par des ouvertures (15) depuis la face supérieure (7) de la plaque, en particulier selon l'une quelconque des revendications 1 à 41,
- dans lequel le cadre (2) et les récipients (3) sont fabriqués dans un procédé de moulage par injection de plusieurs composants,
- dans lequel le cadre (2) d'abord, puis les récipients (3) sont moulés par injection, et
- dans lequel les récipients (3) sont injectés à la suite de l'injection du cadre (2) après écoulement d'un temps assurant à peu près complètement la contraction du cadre (2).

45. Procédé selon la revendication 44, dans lequel les récipients (3) sont moulés chacun par injection depuis un point d'injection propre de leur fond (9).

46. Procédé selon l'une quelconque des revendications 42 à 45, dans lequel le cadre (2) est moulé par injection depuis plusieurs points d'injection dans la zone du bord.
